# EUROPEAN PATENT SPECIFICATION

(11) **EP 3 494 881 B1**
(45) Date of publication and mention of the grant of the patent: **29.04.2020**
(21) Application number: 18208626.4
(22) Date of filing: 27.11.2018
(51) Int. Cl.: A61B 5/107, A61B 18/14, A61F 5/445, A61B 17/00, A61B 17/28, A61B 18/00

(54) **ORGAN MEASURING TOOL ASSEMBLY**
ORGANMESSWERKZEUGANORDNUNG
ENSEMBLE OUTIL DE MESURE D'ORGANES

(30) Priority: 28.11.2017 US 201762591683 P; 25.09.2018 US 201816140577
(43) Date of publication of application: 12.06.2019
(73) Proprietor: Covidien LP, Mansfield, MA 02048 (US)
(72) Inventor: AVVLN, Srinivasa Murthy Aravalli, 500049 Hyderabad (IN)
(74) Representative: Soames, Candida Jane

(56) References cited:
- WO-A1-00/78233
- WO-A1-2013/037915
- WO-A2-2004/082723
- US-A- 3 369 550

## Description

### BACKGROUND

### 1. Technical Description

The present disclosure is directed to an organ measuring tool assembly and, more particularly, to an organ measuring tool assembly including a device for measuring the size of the bowel during an ostomy procedure.

### 2. Background of Related Art

Exteriorization of an internal body vessel such as the intestine is called a stoma. Stomas may be created in conjunction with an ostomy procedure, e.g., colostomy or ileostomy, by suturing a bisected portion of an intestine to the abdominal wall to provide internal access into the intestine for collecting fecal matter. Parastomal herniation is the most significant and frequent complication associated with patient's that have had ostomy procedures. Parastomal herniation occurs due to an improperly formed or sized hole in the abdominal muscles, i.e., the rectus sheath, in the abdominal wall.

Parastomal herniation may result when the hole formed in the rectus sheath is of an improper size. If the size of the hole created in the rectus sheath is too small, the clinician may have to expand the hole to exteriorize the bowel. This hole expansion may allow internal body organs to pass into the expanded hole and cause strangulation of the internal body organs. Similarly, if the hole created in the rectus sheath is too big, strangulation of internal body organs may also occur if body organs pass into the space between the opening and the internal body organ. WO2004/082723 discloses a tool kit of an open approach stapler. US3,369,550 discloses a cryogenic clamp with interchangeable heads on the same instrument. Furthermore, WO00/78233 discloses a surgical clamp with replaceable clamp members.

Thus, a continuing need exists in the art for a device that assists a clinician in properly sizing the hole to be formed in the rectus sheath during an ostomy procedure.

### SUMMARY

The invention is defined by the independent claims 1 and 6. One aspect of the disclosure is directed to an organ measuring tool assembly including a grasping device and a measuring device. The grasping device includes a handle assembly, an elongate body extending distally from the handle assembly, and first and second jaws supported on the elongate body. The first and second jaws are movable in response to actuation of the handle assembly between an open position and a closed position. The measuring device has a coupling portion and a measuring portion. The coupling portion is configured to releasably engage the first and second jaws of the grasping device to releasably couple the measuring device to the grasping device. The measuring device defines a predetermined diameter.

Another aspect of the disclosure is directed to a kit for measuring a diameter of a body organ. The kit includes a grasping device and a plurality of measuring devices. The grasping device has a handle assembly, an elongate body extending distally from the handle assembly, and first and second jaws that are movable in response to actuation of the handle assembly between an open position and a closed position. Each of the plurality of measuring devices has a coupling portion and a measuring portion. The coupling portion is configured to releasably engage the first and second jaws of the grasping device to releasably couple the measuring device to the grasping device. Each of the measuring devices defines a predetermined diameter that is different from the predetermined diameter of the other of the plurality of measuring devices.

In embodiments, the measuring device includes a first measuring component and a second measuring component. The first measuring component is configured to be releasably coupled to the first jaw of the grasping device and the second measuring component is configured to be releasably coupled to the second jaw of the grasping device.

In some embodiments, each of the first and second measuring components includes a coupling portion and a measuring portion that extends distally from the coupling portion.

In certain embodiments, each of the first and second measuring portions has a semi-circular configuration such that the first and second measuring portions define the predetermined diameter when the jaws are in the closed position.

In embodiments, the measuring portions are formed of a resilient material.

In some embodiments, the measuring device includes a ring-shaped body.

In certain embodiments, the ring-shaped body is formed from a resilient material.

In embodiments, the coupling portion includes spaced sleeves that are positioned on an outer periphery of the ring-shaped body.

In some embodiments, the coupling portion defines channels that are configured to receive the first and second jaws of the grasping device.

### BRIEF DESCRIPTION OF THE DRAWINGS

Various embodiments of the presently disclosed bowel measuring tool assembly are described herein below with reference to the drawings, wherein:
FIG. 1 is a side perspective view of a one exemplary embodiment of the presently disclosed bowel measuring tool assembly with the bowel measuring tool assembly in an open position;
FIG. 2 is a side perspective view of a distal end of the bowel measuring tool assembly shown in FIG. 1 with a measuring device of the bowel measuring tool assembly separated from a grasping device of the bowel measuring tool assembly;
FIG. 3 is an enlarged view of the indicated area of detail shown in FIG. 1;
FIG. 4 is a side view of a kit including the grasping device shown in FIG. 1 with a plurality of measuring devices of different sizes with one of the measuring devices supported on the grasping device;
FIG. 5 is a side view of the bowel measuring tool assembly shown in FIG. 1 positioned within a cannula with the bowel measuring tool assembly in a closed position;
FIG. 6 is a side perspective view of the bowel measuring tool assembly shown in FIG. 1 inserted through a cannula with the tool assembly in a closed position and the measuring device positioned about an internal organ of a patient;
FIG. 7 is another exemplary embodiment of a measuring device of the presently disclosed bowel measuring tool assembly;
FIG. 8 is a side view of the measuring device shown in FIG. 7 supported on another embodiment of a grasping device of the presently disclosed bowel measuring tool assembly;
FIG. 9 is a side view of the bowel measuring tool assembly shown in FIG. 8 positioned within a cannula with the bowel measuring tool assembly in a closed position and the measuring device deformed;
FIG. 10 is a front view of a plurality of the measuring devices shown in FIG. 7 having a variety of different sizes;
FIG. 11 is a top view of a stoma stapled to the abdominal wall of a patient; and
FIG. 12 is a side view of the stoma and the abdominal wall.

### DETAILED DESCRIPTION OF EMBODIMENTS

The presently disclosed device will now be described in detail with reference to the drawings in which like reference numerals designate identical or corresponding elements in each of the several views. However, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "endoscopic" is used generally used to refer to endoscopic, laparoscopic, arthroscopic, and/or any other procedure conducted through small diameter incision or cannula. Further, the term "clinician" is used generally to refer to medical personnel including doctors, nurses, and support personnel.

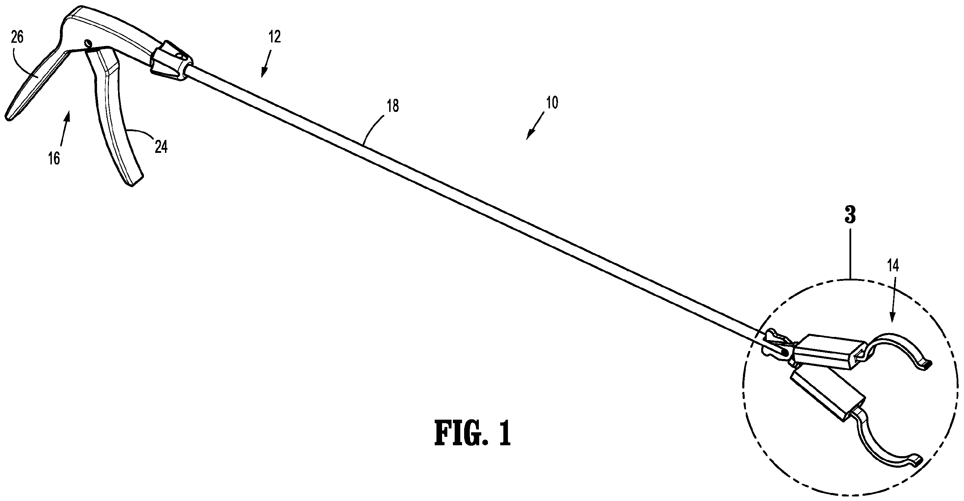

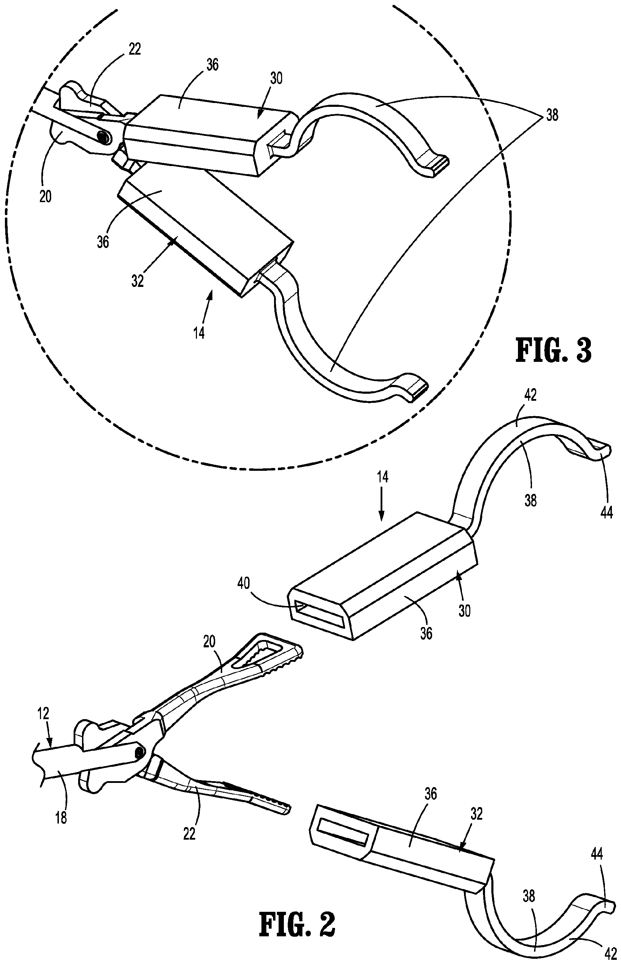

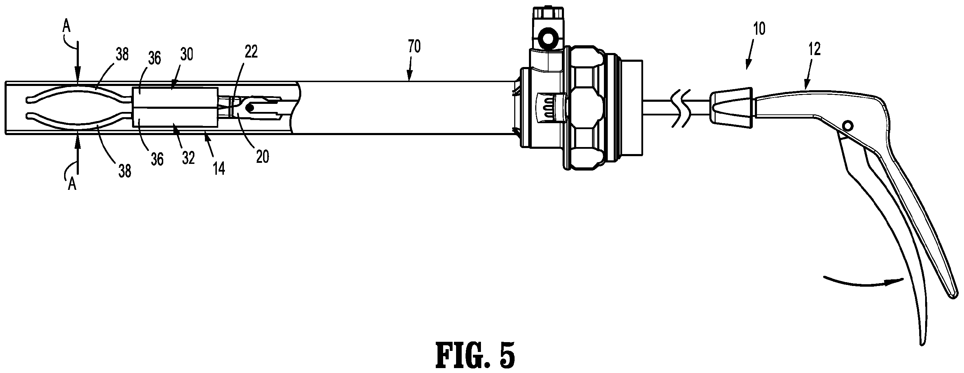

Referring to FIGS. 1-3, the presently disclosed organ measuring tool assembly is shown generally as 10 and includes a grasping device 12 and a measuring device 14 releasably coupled to a distal end of the grasping device 12. The grasping device 12 includes a handle assembly 16, an elongate body portion 18, a first jaw 20, and a second jaws 22 (FIG. 3). The first and second jaws 20, 22 are pivotably supported on a distal end of the elongate body portion 18 in relation to each other between an open position (FIG. 2) and a closed position (FIG. 5.) The handle assembly 16 includes a pivotable trigger 24 and a stationary handle 26. Although not shown, the pivotable trigger 24 is connected to the first and second jaws 20, 22 by an actuator that functions to move the first and second jaws 20, 22 from the open position (FIG. 2) to the closed position (FIG. 5) when the pivotable trigger 24 is moved towards the stationary handle 26. It is envisioned that the grasping device 12 may include any of a variety of different mechanisms for opening and closing the first and second jaws 20, 22.

The measuring device 14 includes a first measuring component 30 that is releasably coupled to the first jaw 20 and a second measuring component 32 that is releasably coupled to the second jaw 22. Each of the measuring components 30, 32 includes a coupling portion 36 and a measuring portion 38. The coupling portion 36 of the first and second measuring components 30, 32 is configured to releasably engage one of the jaws 20, 22. In embodiments, the coupling portion 36 defines a channel 40 (FIG. 2) that is dimensioned to receive one of the first and second jaws 20, 22 to secure the measuring component 30, 32 on the respective jaw 20, 22. In embodiments, the coupling portion 36 can be deformable and/or resilient to allow the channel 40 to expand and frictionally engage the respective jaw 20, 22. Alternately, other coupling devices or configurations can be provided to secure the measuring components 30, 32 to the first and second jaws 20, 22.

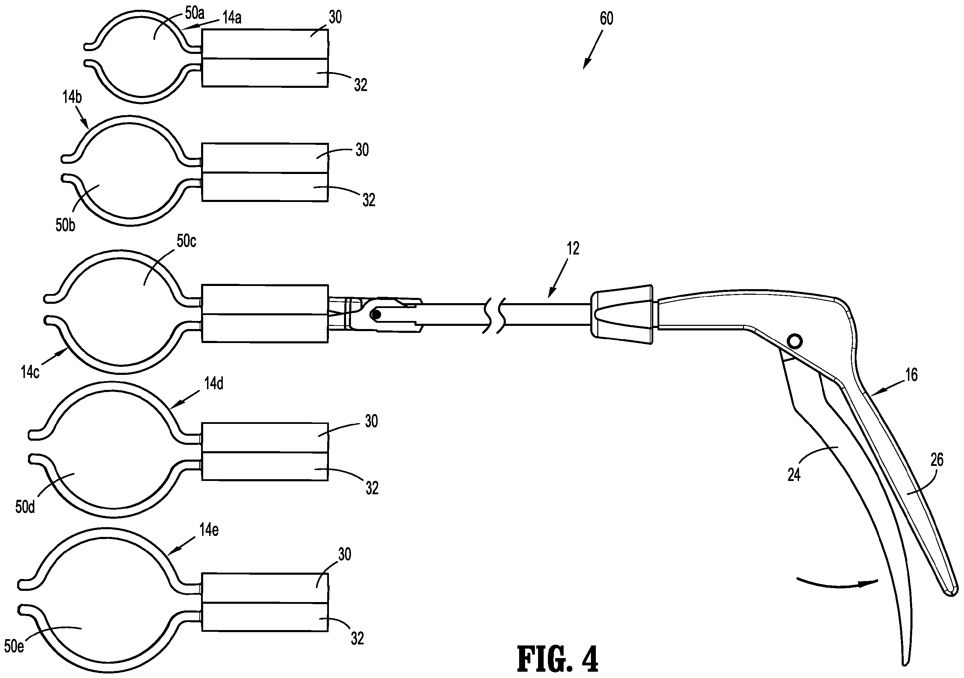

Each of the measuring portions 38 extends distally from a distal portion of the coupling portion 36 and includes a semi-circular body portion 42 having a linear distal extension 44. It envisioned that the body portions 42 need not have a linear extension 44. When the first and second jaws 20 and 22 of the grasping device 12 are moved from an open position (FIG. 1) to a closed position (FIG. 4), the semi-circular body portions 42 of the measuring portions 38 of the measuring components 30, 32 are positioned adjacent to one another to define a circular opening 50a-e (FIG. 4) of a predetermined diameter "D".

Referring to FIG. 4, a kit 60 may be provided that includes the grasping device 12 and a plurality of measuring devices 14a-e. In embodiments, each of the plurality of measuring devices has a different predetermined diameter and can be selectively attached to the jaws 20, 22 of the grasping device 12 to identify the diameter of a particular body organ, such as an intestine or colon of a patient. In embodiments, the diameter of the measuring devices 14a-e may be 21mm, 25mm, 28mm, 31mm, and to 33mm, respectively. Alternately, the diameter of the measuring devices 14a-e may be selected as needed for a particular surgical procedure or a particular patient. In embodiments, the diameter of each of the measuring devices 14a-e can be written on the measuring device 14a-e to identify to the clinician the diameter of the particular measuring device 14a-e.

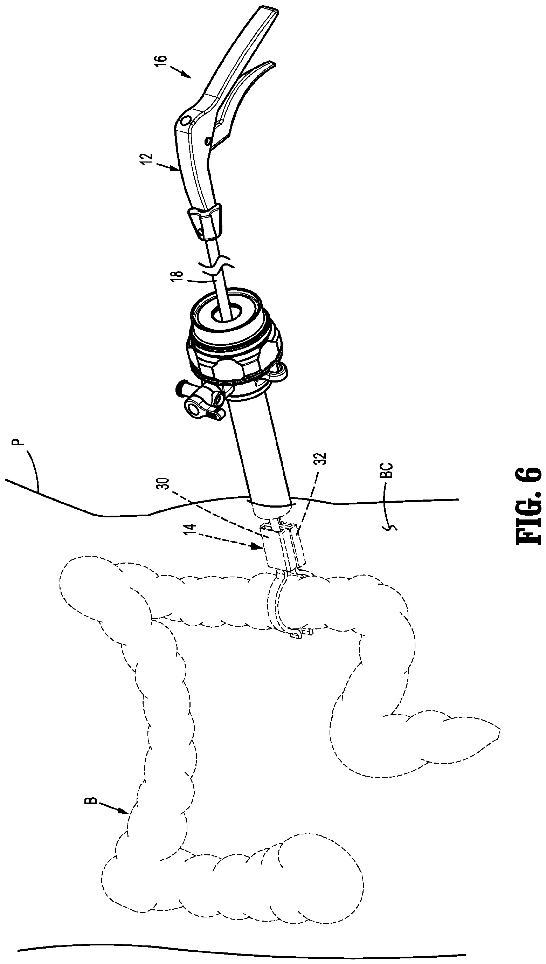

Referring to FIGS. 5 and 6, in use, the measuring device 14a-e is attached to the jaws 20, 22 of the grasping device 12 and the jaws 20, 22 of the grasping device 12 are moved to the closed position. With the jaws 20, 22 of the grasping device 12 in the closed position, the jaws 20, 22 and the measuring device 14 including the measuring components 30, 32 are inserted through the cannula 70 into a body cavity "BC" (FIG. 6) of patient "P" such that the measuring device 14a-e is positioned adjacent a body organ, e.g., the bowel "B". As used herein, the "bowel" includes the small and large intestines, the colon, and the rectum. As discussed above, the measuring portions 38 of the measuring components 30, 32 of the measuring devices 14a-e are formed of a flexible and/or resilient material. This allows the measuring portions 38 of each of the measuring components to be deformed inwardly in the direction indicated by arrows "A" in FIG. 5 to allow the measuring device 14 of the organ measuring tool assembly 10 to be inserted through a smaller diameter cannula, e.g., a 10mm cannula, into the body cavity "BC".

After one of the measuring devices 14a-e of the organ measuring tool assembly 10 is inserted through the cannula 70 into the body cavity "BC" (FIG. 6) of the patient "P", the first and the second jaws 20, 22 of the grasper 12 can be opened and positioned about the bowel "B" and subsequently moved to the closed position about the bowel "B" to determine the size of the bowel "B". If the selected measuring device 14a-e has a larger or smaller diameter than the bowel "B", the measuring device 14a-e of the organ measuring tool assembly 10 can be removed from the cannula 70 and replaced with a measuring device 14a-e having a smaller or larger diameter to approximate the diameter of the bowel "B". Once the diameter of the measuring device 14a-e attached to the grasping device 12 closely matches or approximates the size of the bowel "B", the selected measuring device 14a-e of the organ measuring tool assembly 10 can be withdrawn from the cannula 70 and the size of the "bowel" can be determined by identifying the size of the selected measuring device 14a-e.

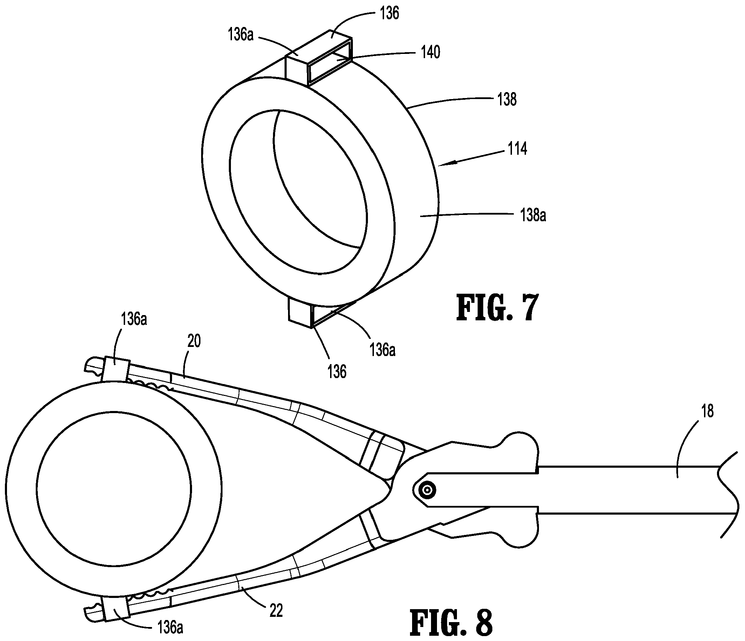

Referring to FIGS. 7 and 8, in an alternate embodiment of the presently disclosed measuring device shown generally as 114, the measuring device 114 includes a coupling portion 136 and a measuring portion 138. The measuring portion 138 includes a circular or ring-shaped body 138a that is formed of a resilient material. The coupling portion 136 includes a pair of spaced sleeves 136a that are positioned on an outer periphery of the ring-shaped body 138a. In embodiments, each of the sleeves 136a defines a channel 140 that is dimensioned to receive a distal end of one of the first and second jaws 20, 22. The coupling portions 136 of the measuring device 114 can be formed of a resilient material that is stretched about the jaws 20, 22 as the jaws 20, 22 are inserted through the channels 140 of the coupling portions 136 to firmly secure the coupling portions 136 to the jaws 20, 22 of the grasping device 12.

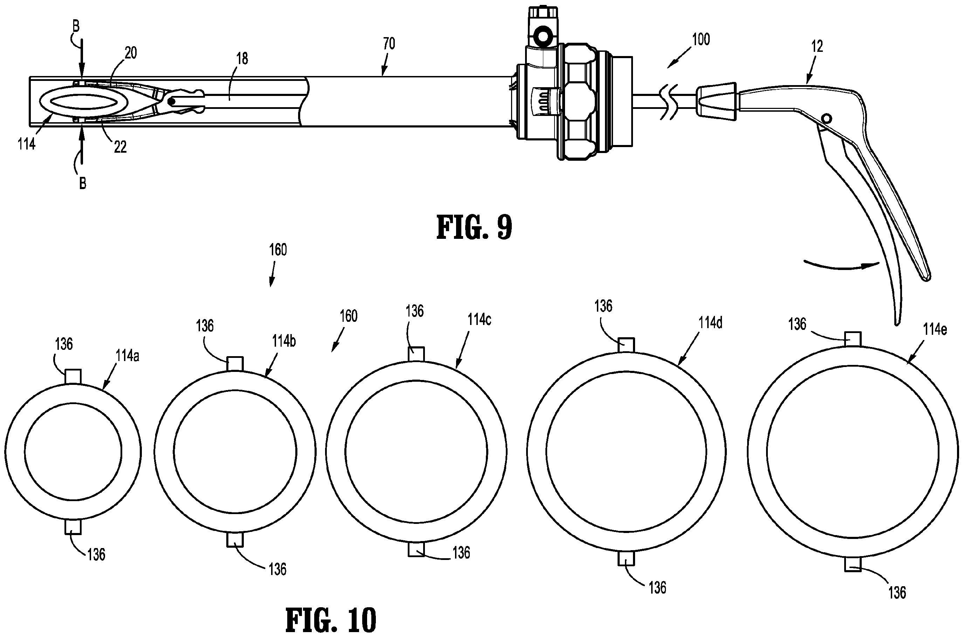

Referring to FIGS. 9 and 10, a kit 160 may be provided that includes the grasping device 12 (FIG. 9) and a plurality of measuring devices 114a-e (FIG. 10) that have different predetermined diameters and can be selectively attached to the jaws 20, 22 of the grasping device 12 to identify the diameter of a particular body organ such as the bowel of a patient. In embodiments, the diameter of the measuring devices 114a-e may be 21mm, 25mm, 28mm, 31mm, and to 33mm, respectively. Alternately, the diameter of the measuring devices 114a-e may be selected as needed for a particular surgical procedure or a particular patient. In embodiments, the diameter of each of the measuring devices 114a-e can be written on the measuring device 114a-e to identify to the clinician the diameter of the particular measuring device 14a-e.

In use, the organ measuring tool assembly 100, which includes the grasping device 12 and a selected one of the measuring devices 114a-e, is inserted through the cannula 70 into a body cavity of patient such that the respective measuring device 114a-e is positioned adjacent a body organ. As discussed above, the ring-shaped body 138a of the respective measuring device 114a-e is formed of a flexible and/or resilient material. This allows the measuring device 114a-e to be deformed inwardly in the direction indicated by arrows "B" in FIG. 9 when the jaws 20, 22 of the grasping device 12 are closed to allow the measuring device 114a-e of the organ measuring tool assembly 100 to be inserted through a smaller diameter cannula 70, e.g., a 10mm cannula.

After the organ measuring tool assembly 100 is inserted through the cannula 70 into a body cavity of a patient, the first and the second jaws 20, 22 of the grasper device 12 can be positioned adjacent a body organ, e.g., the bowel, and the jaws 20, 22 of the grasping device 12 can be moved to the open position (FIG. 8). Movement of the jaws 20, 22 of the grasping device 12 allows the respective measuring device 114a-e to return to an undeformed configuration. In the undeformed configuration, a body organ can be inserted through the respective measuring device 114a-e to allow a clinician to identify the size or diameter of the body organ. It is noted that the body organ can only be inserted through the respective measuring device 114a-e after the body organ has been transected such as during a colostomy procedure to remove a section of the colon. If the measuring device 114 has a larger or smaller diameter than the body organ inserted there through, the organ measuring tool assembly 100 can be removed from the cannula 70 and the measuring device 114 can be replaced with a measuring device having a smaller or larger diameter that more closely matches or approximates the diameter of the body organ. Once the diameter of the measuring device 114 attached to the grasping device 12 closely matches or approximates the size of the body organ, the organ measuring tool assembly 100 can be withdrawn from the cannula 70 and the size or diameter of the body organ can be identified by a clinician such as by reading the size or diameter of the attached measuring device 114.

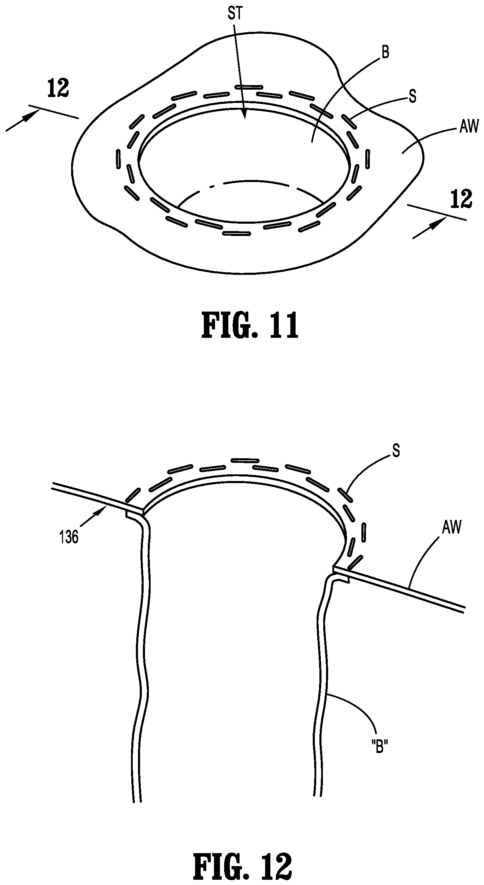

Referring to FIGS. 11 and 12, after determining the diameter of a body organ, e.g., the bowel "B", a clinician can use this diameter to determine the appropriate size hole to be formed in the abdominal wall "AW" and the rectus sheath (not shown). In embodiments, the hole can be formed with a circular stapler which provides an annular array of reinforcing staples "S" about the hole. The staples "S" can also be used to secure the "B" to the abdominal wall "AW" to form the stoma "ST".

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

## Claims

1. An organ measuring tool assembly (100) comprising:
a grasping device (12) having a handle assembly (16), an elongate body (18) extending distally from the handle assembly (16), and first and second jaws (20, 22) supported on the elongate body (18), the first and second jaws (20, 22) being movable in response to actuation of the handle assembly (16) between an open position and a closed position; and
a measuring device (114) having a coupling portion (136) and a measuring portion (138), **characterised in that** the measuring portion (138) comprises a circular or ring-shaped body (138a) that is formed of a resilient material, and the coupling portion (136) being configured to releasably engage the first and second jaws (20, 22) of the grasping device (12) to releasably couple the measuring device (114) to the grasping device (12), wherein the measuring device (114) defines a predetermined diameter, wherein the coupling portion (136) includes a pair of spaced sleeves (136a) positioned on an outer periphery of the circular or ring-shaped body (138a).

2. An organ measuring tool assembly (100) according to claim 1, wherein said sleeves are configured to receive a distal end of the first and second jaws (20, 22) of the grasping device (12).

3. An organ measuring tool assembly (100) according to claim 2, wherein each of the spaced sleeves (136a) defines a channel (14) that is dimensioned to receive a distal end of one of the first or second jaws (20, 22).

4. An organ measuring tool assembly (100) according to any preceding claim, wherein the coupling portion (136) is formed of a resilient material.

5. An organ measuring tool assembly (100) according to any preceding claim, further comprising a plurality of measuring devices (114a-e) that have different predetermined diameters, wherein said measuring devices (114a-e) can each be selectively attached to the jaws (20, 22)

6. A kit (160) for measuring a diameter of a body organ, the kit comprising:
a grasping device (12) having a handle assembly (16), an elongate body (18) extending distally from the handle assembly (16), and first and second jaws (20, 22) supported on the elongate body (18), the first and second jaws (20, 22) being movable in response to actuation of the handle assembly (16) between an open position and a closed position; and
a plurality of measuring devices (114a-e), each of the plurality of measuring devices (114a-e) having a coupling portion (136) and a measuring portion (138), **characterised in that** the measuring portion (138) of each measuring device (114a-e) comprises a circular or ring-shaped body (138a) that is formed of a resilient material, and wherein the coupling portion (136) is configured to releasably engage the first and second jaws (20, 22) of the grasping device (12) to releasably couple the measuring devices (114a-e) to the grasping device (12), wherein each of the measuring devices (114a-e) defines a predetermined diameter that is different from the predetermined diameter of the other of the plurality of measuring devices (114a-e), wherein the coupling portion (136) of each measuring device includes a pair of spaced sleeves (136a) positioned on an outer periphery of the circular or ring-shaped body (138a).

7. The kit of claim 6, wherein said sleeves are configured to receive a distal end of the first and second jaws (20, 22) of the grasping device (12).

8. The kit of claim 7, wherein each of the spaced sleeves (136a) of the coupling portion (136) defines a channel (14) that is dimensioned to receive a distal end of one of the first or second jaws (20, 22).

9. The kit according to any of claims 6-8, wherein the coupling portion (136) is formed of a resilient material.

## Patentansprüche

1. Organmesswerkzeuganordnung (100), umfassend:
eine Greifvorrichtung (12) mit einer Griffanordnung (16), einem länglichen Körper (18), der sich distal von der Griffanordnung (16) erstreckt, und einer ersten und einer zweiten Backe (20, 22), die auf dem länglichen Körper (18) gelagert sind, wobei die erste und die zweite Backe (20, 22) als Reaktion auf die Betätigung der Griffanordnung (16) zwischen einer offenen Position und einer geschlossenen Position bewegbar sind; und
eine Messvorrichtung (114) mit einem Kopplungsabschnitt (136) und einem Messabschnitt (138), **dadurch gekennzeichnet, dass** der Messabschnitt (138) einen kreis- oder ringförmigen Körper (138a) umfasst, der aus einem elastischen Material gebildet ist, und wobei der Kopplungsabschnitt (136) so konfiguriert ist, dass er lösbar mit der ersten und der zweiten Backe (20, 22) der Greifvorrichtung (12) in Eingriff kommt, um die Messvorrichtung (114) lösbar mit der Greifvorrichtung (12) zu koppeln, wobei die Messvorrichtung (114) einen vorbestimmten Durchmesser definiert, wobei der Kopplungsabschnitt (136) ein Paar beabstandeter Hülsen (136a) umfasst, die an einem Außenumfang des kreis- oder ringförmigen Körpers (138a) positioniert sind.

2. Organmesswerkzeuganordnung (100) nach Anspruch 1, wobei die Hülsen zur Aufnahme eines distalen Endes der ersten und zweiten Backe (20, 22) der Greifvorrichtung (12) konfiguriert sind.

3. Organmesswerkzeuganordnung (100) nach Anspruch 2, wobei jede der beabstandeten Hülsen (136a) einen Kanal (14) definiert, der zur Aufnahme eines distalen Endes einer der ersten oder zweiten Backen (20, 22) bemessen ist.

4. Organmesswerkzeuganordnung (100) nach einem beliebigen vorhergehenden Anspruch, wobei der Kopplungsabschnitt (136) aus einem elastischen Material gebildet ist.

5. Organmesswerkzeuganordnung (100) nach einem beliebigen vorhergehenden Anspruch, ferner umfassend mehrere Messvorrichtungen (114a-e), die unterschiedliche vorbestimmte Durchmesser aufweisen, wobei die Messvorrichtungen (114a-e) jeweils selektiv an den Backen (20, 22) angebracht werden können.

6. Kit (160) zum Messen eines Durchmessers eines Körperorgans, wobei das Kit Folgendes umfasst:
eine Greifvorrichtung (12) mit einer Griffanordnung (16), einem länglichen Körper (18), der sich distal von der Griffanordnung (16) erstreckt, und einer ersten und einer zweiten Backe (20, 22), die auf dem länglichen Körper (18) gelagert sind, wobei die erste und die zweite Backe (20, 22) als Reaktion auf die Betätigung der Griffanordnung (16) zwischen einer offenen Position und einer geschlossenen Position bewegbar sind; und
mehrere Messvorrichtungen (114a-e), wobei jede der mehreren Messvorrichtungen (114a-e) einen Kopplungsabschnitt (136) und einen Messabschnitt (138) aufweist, **dadurch gekennzeichnet, dass** der Messabschnitt (138) jeder Messvorrichtung (114a-e) einen kreis- oder ringförmigen Körper (138a) umfasst, der aus einem elastischen Material gebildet ist, und wobei der Kopplungsabschnitt (136) so konfiguriert ist, dass er lösbar mit der ersten und der zweiten Backe (20,22) der Greifvorrichtung (12) in Eingriff kommt, um die Messvorrichtungen (114a-e) lösbar mit der Greifvorrichtung (12) zu koppeln, wobei jede der Messvorrichtungen (114a-e) einen vorbestimmten Durchmesser definiert, der sich von dem vorbestimmten Durchmesser der anderen der mehreren Messvorrichtungen (114a-e) unterscheidet, wobei der Kopplungsabschnitt (136) jeder Messvorrichtung ein Paar beabstandeter Hülsen (136a) umfasst, die an einem Außenumfang des kreis- oder ringförmigen Körpers (138a) positioniert sind.

7. Kit nach Anspruch 6, wobei die Hülsen so konfiguriert sind, dass sie ein distales Ende der ersten und zweiten Backe (20, 22) der Greifvorrichtung (12) aufnehmen.

8. Kit nach Anspruch 7, wobei jede der beabstandeten Hülsen (136a) des Kopplungsabschnitts (136) einen Kanal (14) definiert, der zur Aufnahme eines distalen Endes einer der ersten oder zweiten Backen (20, 22) bemessen ist.

9. Kit nach einem der Ansprüche 6 bis 8, wobei der Kopplungsabschnitt (136) aus einem elastischen Material gebildet ist.

## Revendications

1. Ensemble d'outils de mesure d'organes (100) comprenant :
un dispositif de préhension (12) présentant un ensemble poignée (16), un corps allongé (18) s'étendant de manière distale à partir de l'ensemble poignée (16) et des première et seconde mâchoires (20, 22) supportées sur le corps allongé (18), les première et seconde mâchoires (20, 22) pouvant être déplacées en réponse à l'actionnement de l'ensemble poignée (16) entre une position ouverte et une position fermée ; et
un dispositif de mesure (114) présentant une partie d'accouplement (136) et une partie de mesure (138), **caractérisé en ce que** la partie de mesure (138) comprend un corps circulaire ou en forme d'anneau (138a) qui est constitué d'un matériau élastique, et la partie d'accouplement (136) étant conçue pour entrer en prise de manière amovible les première et seconde mâchoires (20, 22) du dispositif de préhension (12) pour accoupler de manière amovible le dispositif de mesure (114) au dispositif de préhension (12), dans lequel le dispositif de mesure (114) définit un diamètre prédéfini, dans lequel la partie d'accouplement (136) comprend une paire de manchons espacés (136a) positionnés sur une périphérie extérieure du corps circulaire ou en forme d'anneau (138a).

2. Ensemble d'outils de mesure d'organes (100) selon la revendication 1, dans lequel lesdits manchons sont conçus pour recevoir une extrémité distale des première et deuxième mâchoires (20, 22) du dispositif de préhension (12).

3. Ensemble d'outils de mesure d'organes (100) selon la revendication 2, dans lequel chacun des manchons espacés (136a) définit un canal (14) qui est dimensionné pour recevoir une extrémité distale de l'une parmi les première ou deuxième mâchoires (20, 22).

4. Ensemble d'outils de mesure d'organes (100) selon l'une quelconque des revendications précédentes, dans lequel la partie d'accouplement (136) est constituée d'un matériau élastique.

5. Ensemble d'outils de mesure d'organes (100) selon l'une quelconque des revendications précédentes, comprenant en outre une pluralité de dispositifs de mesure (114a-e) qui ont des diamètres prédéfinis différents, dans lequel lesdits dispositifs de mesure (114a-e) peuvent chacun être fixés de manière sélective aux mâchoires (20, 22).

6. Kit (160) destiné à mesurer le diamètre d'un organe corporel, le kit comprenant :
un dispositif de préhension (12) présentant un ensemble poignée (16), un corps allongé (18) s'étendant de manière distale à partir de l'ensemble poignée (16) et des première et seconde mâchoires (20, 22) supportées sur le corps allongé (18), les première et seconde mâchoires (20, 22) pouvant être déplacées en réponse à l'actionnement de l'ensemble poignée (16) entre une position ouverte et une position fermée ; et
une pluralité de dispositifs de mesure (114a-e), chacun de la pluralité de dispositifs de mesure (114a-e) présentant une partie d'accouplement (136) et une partie de mesure (138), **caractérisé en ce que** la partie de mesure (138) de chaque dispositif de mesure (114a-e) comprend un corps circulaire ou en forme d'anneau (138a) qui est constitué d'un matériau élastique, et dans lequel la partie d'accouplement (136) est conçue pour entrer en prise de manière amovible les première et deuxième mâchoires (20, 22) du dispositif de préhension (12) pour accoupler de manière amovible les dispositifs de mesure (114a-e) au dispositif de préhension (12), dans lequel chacun des dispositifs de mesure (114a-e) définit un diamètre prédéfini qui est différent du diamètre prédéfini de l'autre parmi la pluralité de dispositifs de mesure (114a-e), dans lequel la partie d'accouplement (136) de chaque dispositif de mesure comprend une paire de manchons espacés (136a) positionnés sur une périphérie extérieure du corps circulaire ou en forme d'anneau (138a).

7. Kit selon la revendication 6, dans lequel lesdits manchons sont conçus pour recevoir une extrémité distale des première et deuxième mâchoires (20, 22) du dispositif de préhension (12).

8. Kit selon la revendication 7, dans lequel chacun des manchons espacés (136a) de la partie d'accouplement (136) définit un canal (14) qui est dimensionné pour recevoir une extrémité distale de l'une parmi les première ou seconde mâchoires (20, 22).

9. Kit selon l'une quelconque des revendications 6 à 8, dans lequel la partie d'accouplement (136) est constituée d'un matériau élastique.
